Europäisches Patentamt

European Patent Office

Office européen des brevets

(11) Veröffentlichungsnummer: **0 471 335 A1**

## EUROPÄISCHE PATENTANMELDUNG

(21) Anmeldenummer: **91113568.9**

(22) Anmeldetag: **13.08.91**

(51) Int. Cl.⁵: **A61M 5/24**, A61M 5/315

(30) Priorität: **14.08.90 DE 4025717**

(43) Veröffentlichungstag der Anmeldung:
**19.02.92 Patentblatt 92/08**

(84) Benannte Vertragsstaaten:
**BE DE DK ES FR GB GR IT LU NL**

(71) Anmelder: **HOECHST AKTIENGESELLSCHAFT**
**Postfach 80 03 20**
**W-6230 Frankfurt am Main 80(DE)**

(72) Erfinder: **Loos, Hans-Joachim**
**An der Schleuse 5**
**W-6095 Ginsheim-Gustavsburg(DE)**
Erfinder: **Ziegert, Günter**
**Loreleistrasse 34**
**W-6230 Frankfurt am Main 80(DE)**

(54) **Spritzengestell.**

(57) Bei dem Spritzengestell für Zylinderampullen mit
einer für die Aufnahme der Zylinderampulle (3) vorgesehenen Aufnahmeeinrichtung (11) und einem
Griff (1) mit Bohrung zur Aufnahme einer längsverschiebbaren Kolbenstange (12) mit Bestätigungseinrichtung (13) besteht die Aufnahmeeinrichtung (11)
aus einem U-förmigen Kragen (17) mit umlaufender
Nut (2) in seiner Wurzel.

Fig. 1

EP 0 471 335 A1

Die Erfindung betrifft ein Spritzengestell für Zylinderampullen mit einer Aufnahmeeinrichtung zur Aufnahme der Zylinderampullen und einem Griff mit Bohrung zur Aufnahme einer längsverschiebbaren Kolbenstange mit Betätigungseinrichtung.

Spritzengestelle der genannten Art sind aus der DE-PS 29 15 338 bekannt und zeichnen sich dadurch aus, daß die Ampulle in einer Hülse gehalten ist, die an ihrem vorderen Ende eine Kanüle aufnehmen kann und an ihrem hinteren Ende einen Griff mit Bohrungen aufweist, die für die Aufnahme einer mit Betätigungseinrichtung versehenen Kolbenstange bestimmt ist. Die Ampulle ist einerseits durch einen als Kolben wirkenden Stopfen verschlossen, in den die Kolbenstange eingreift und andererseits durch eine Gummimembran mit Metallkappe. Dieses Spritzengestell ist aufwendig in der Herstellung, der Bedienung und Wartung.

Hier will die Erfindung Abhilfe schaffen. Die Erfindung wie sie in den Ansprüchen beschrieben ist, löst die Aufgabe dadurch, daß die Aufnahmeeinrichtung für die Ampulle aus einem U-förmigen Kragen mit einer umlaufenden Nut in seiner Wurzel besteht.

Zur Aufnahme von Zylinderampullen ohne Flansch am Stopfen verschlossenen Ende kann der Kragen mit umlaufender Nut einen Adapter aufweisen, der als U-förmige Rinne ausgebildet ist, die an einem Ende mit einem Flansch und am anderen Ende mit einer Haube versehen ist.

Die Vorteile der Erfindung sind im wesentlichen in der einfacheren Bedienung und Wartung (kein Autoklavieren des Spritzengestells) zu sehen.

Im folgenden wird die Erfindung anhand von lediglich einen Ausführungsweg zeigenden Zeichnungen näher erläutert. Es zeigt

Figur 1 das Spritzengestell mit Zylinderampulle und Einmalkanüle mit Schutzkappe;

Figur 2 einen Adapter teilweise geschnitten und

Figur 3 eine Zylinderampulle ohne Flansch.

Figur 4 einen vergrößerten Teilschnitt gemäß der Linie IV-IV in Figur 1.

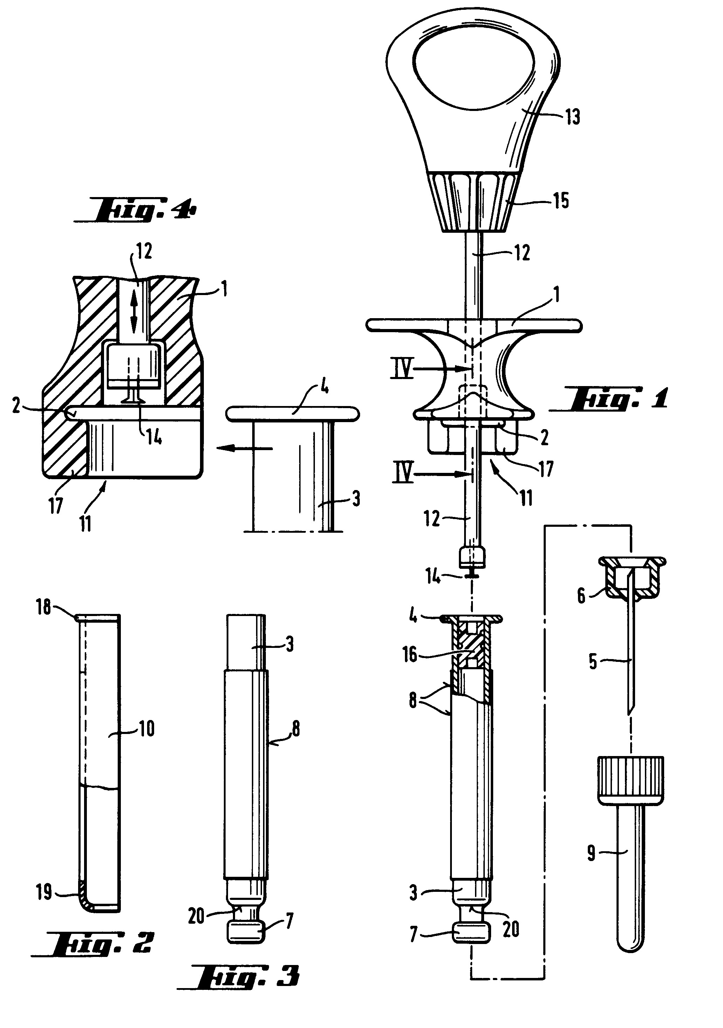

Die bei dem Spritzengestell vorgesehenen Aufnahmeeinrichtung 11 ist integrales Teil des Griffes 1, der mit einer Bohrung zur Aufnahme einer längsverschiebbaren Kolbenstange 12 mit Betätigungseinrichtung 13 versehen ist. In der Kolbenstange 12 sind Krallen 14 angeordnet, die mittels Manipulator 15 gehandhabt werden. Mit den Krallen 14 kann die Kolbenstange formschlüssig mit dem als Kolben wirkenden Stopfen 16, der die Ampulle 3 hält, verbunden werden. Die Aufnahmeeinrichtung 11 für die Ampulle 3 besteht aus einem U-förmigen Kragen 17 mit einer umlaufenden Nut 2 in seiner Wurzel. Die Öffnung des Kragens 17 entspricht dem Durchmesser der Ampulle 3, die mit ihrem umlaufenden Flansch 4 in die Nut 2 eingreift. Für Ampullen ohne Flansch ist für die Aufnahmeeinrichtung 11 ein Adapter 10 vorgesehen, der als Rinne mit U-förmigem Querschnitt mit Flansch 18 und Haube 19 ausgebildet ist. Die Ampulle 3 stützt sich mit ihrer Schulter 20 in der Haube 19 ab. 8 deutet ein Folienetikett als Splitterschutz an. Auf den Folienkopf 7 wird die Arretierkappe 6 mit Kanüle 5 geschoben, die hinter dem Ampullenkopf 7 einrastet. Die Kanüle 5 ist mit einer Schutzhaube 9 versehen. Die Kolbenstange 12 kann zusätzlich mit einer Dosierskalierung und Dosiereinstellvorrichtung ausgerüstet werden (nicht dargestellt). Das Spritzengestell kann sowohl für Zylinderampullen als auch für Einmalspritzen verwendet werden. Das Spritzengestell ist für Injektionen jeglicher Art aber auch für die Entnahme von Blut sowie für die Entleerung von Pasten- und Salbenkartuschen geeignet.

## Patentansprüche

1. Spritzengestell für Zylinderampullen mit einer für die Aufnahme der Zylinderampulle vorgesehenen Aufnahmeeinrichtung und einem Griff mit Bohrung zur Aufnahme einer längsverschiebbaren Kolbenstange mit Betätigungseinrichtung, dadurch gekennzeichnet, daß die Aufnahmeeinrichtung 11 aus einem U-förmigen Kragen (17) mit umlaufender Nut (2) in seiner Wurzel besteht.

2. Spritzengestell nach Anspruch 1, dadurch gekennzeichnet, daß der Kragen (17) mit umlaufender Nut (2) einen Adapter (10) zur Aufnahme der Ampulle aufweist, der als U-förmige Rinne ausgebildet ist, die an einem Ende mit einem Flansch (18) und am anderen Ende mit einer Haube (19) versehen ist.

*Fig. 4*

*Fig. 1*

*Fig. 2*

*Fig. 3*

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | EP-A-0 100 369 (SUWANDSCHIEFF) <br> * Seite 14, Zeile 26 - Seite 15, Zeile 8; Abbildungen 7,8 * | 1 | A61M5/24 <br> A61M5/315 |
| Y | --- | 2 | |
| Y | FR-A-1 151 049 (SOCIETE INDUSTRIELLE DE THERAPEUTIQUE SCIENTIFIQUE APPLIQUEE) <br> * Spalte 2, Zeile 14 - Zeile 16; Abbildungen 1-3 * | 2 | |
| | ----- | | |
| | | | **RECHERCHIERTE SACHGEBIETE (Int. Cl.5)** |
| | | | A61M |

Der vorliegende Recherchenbericht wurde für alle Patentansprüche erstellt

| Recherchenort | Abschlußdatum der Recherche | Prüfer |
|---|---|---|
| DEN HAAG | 30 OKTOBER 1991 | SEDY, R. |